# EUROPÄISCHE PATENTSCHRIFT

(11) **EP 3 409 385 B1**
(45) Veröffentlichungstag und Bekanntmachung des Hinweises auf die Patenterteilung: **28.07.2021**
(21) Anmeldenummer: 18174842.7
(22) Anmeldetag: 29.05.2018
(51) Int. Cl.: B08B 11/02, B08B 13/00, B08B 3/02, A61L 2/26

(54) **VORRICHTUNG UND VERFAHREN ZUR BEHANDLUNG VON ATEMSCHUTZMASKEN**
DEVICE AND METHOD FOR TREATING RESPIRATORY MASKS
DISPOSITIF ET PROCÉDÉ DE MANIPULATION DE MASQUES DE PROTECTION RESPIRATOIRE

(30) Priorität: 02.06.2017 DE 102017005285
(43) Veröffentlichungstag der Anmeldung: 05.12.2018
(73) Patentinhaber: Dräger Safety AG & Co. KGaA, 23560 Lübeck (DE)
(72) Erfinder: Hack, Hauke, 23879 Mölln (DE)
(74) Vertreter: Heinemeyer, Karsten

(56) Entgegenhaltungen:
- EP-A1- 2 511 206
- WO-A1-2014/076026
- DE-A1- 2 418 627
- DE-A1- 10 020 835
- DE-B3-102005 033 618
- DE-U1- 8 602 043
- GB-A- 2 122 566

## Beschreibung

Die Erfindung betrifft eine Vorrichtung und ein Verfahren zur Behandlung von Atemschutzmasken, wobei als Behandlung oder im Rahmen der Behandlung insbesondere eine Reinigung der Atemschutzmasken erfolgt.

Persönliche Schutzausrüstung, insbesondere Schutzausrüstung in Form einer Atemschutzmaske, muss nach der Benutzung gereinigt und desinfiziert werden, um eine hygienische weitere Nutzung zu gewährleisten. Es gibt verschiedene Möglichkeiten der Reinigung. Üblich ist die Handwäsche, die Reinigung in Trommelwaschmaschinen oder in Spülautomaten ähnlich Geschirrspülern. Diese genannten Verfahren haben den Nachteil, dass die Atemschutzmasken nach dem Reinigungsgang von Hand in einen Trockenschrank gelegt werden müssen. Außerdem müssen die Masken ausgeschüttet werden, um größere Mengen Wasser zu entfernen. Es sind allerdings bereits diverse Vorrichtungen in Form von Reinigungskabinen oder dergleichen bekannt, mittels derer Atemschutzmasken gereinigt und anschließend ohne Umsetzen getrocknet werden. Für solche Reinigungskabinen gibt es verschiedene Aufnahmegestelle.

Aus der GB 2 122 566 ist eine Vorrichtung geeignet zur Behandlung von Atemschutzmasken gemäß dem Oberbegriff des Anspruchs 1 bekannt.

Eine Vorrichtung zur Behandlung von Atemschutzmasken ist zum Beispiel aus der DE 100 20 835 A1 bekannt. Die dortige Vorrichtung umfasst eine Kabine mit einem um eine vertikale Achse drehbaren Gestell für eine Mehrzahl von Atemschutzmasken. Für jede Atemschutzmaske ist ein um eine horizontale Achse drehbares Aufnahmemodul vorgesehen. Die Anbringung einer Atemschutzmaske an einem Aufnahmemodul erfolgt mittels des Atemschutzgeräteanschlusses der Atemschutzmaske. Dies hat den Nachteil, dass der Anschluss nur bedingt gereinigt wird. Außerdem müssen verschiedene Aufnahmeadapter für verschiedene Anschlussgewinde bereitgehalten werden. Zudem führt die Aufnahme am Atemschutzgeräteanschluss (Lungenautomatenanschluss) möglicherweise zu dessen Überbelastung, sodass die Maske dauerhaft beschädigt wird. Außerdem ist die Bestückung des Gestells aufwendig, da jeweils erst der Adapter mit der Atemschutzmaske verbunden wird und dann in einem zweiten Schritt der Adapter und die Atemschutzmaske mit dem Gestell verbunden wird. Schließlich hängt die Bebänderung der Masken vom Gestell herab, sodass sich die Bebänderung einer Maske mit der Bebänderung einer anderen Maske verschlingen und/oder sich die Bebänderung einer Maske zum Beispiel an dem Gestell verfangen kann.

Aus der DE 200 03 744 U1 ist ebenfalls eine Vorrichtung zur Behandlung von Atemschutzmasken bekannt. Diese umfasst in einem verschließbaren Gehäuse ein Traggestell mit zugeordnetem Düsensystem und einzelnen Behandlungsplätzen.

Aus der DE 10 2005 033 618 B3 ist eine weitere Vorrichtung zur Behandlung von Atemschutzmasken bekannt. Diese umfasst als an einem Träger angeordnete Aufnahme für mindestens eine Atemschutzmaske einen beweglich an dem Träger angelenkten Aufnahmekorb. An einer Innenseite des Aufnahmekorbs befindet sich ein Bürsteneinsatz und durch eine Bewegung des Aufnahmekorbs findet eine Bebürstung der Atemschutzmaske statt. Die Anordnung der Atemschutzmaske in einem Aufnahmekorb hat den Nachteil, dass ein Sprühstrahl eine Atemschutzmaske oftmals nicht oder nur unvollständig erreicht, sodass das Reinigungsergebnis nicht zufriedenstellend ist.

Aus der DE 10 2010 029 221 A1 ist eine Vorrichtung zur Reinigung von gasführenden Elementen und Zubehörteilen eines Atemgeräts bekannt.

Aus der WO 2014/076026 A1 ist schließlich ein Halterungssortiment und ein Reinigungsgerät zur Reinigung von Atemgeräten bekannt, welches in Form eines Gestells mit Haltebügeln für Atemschutzmasken sowie mit Haltebügeln für Innenmasken realisierte Masken-Haltevorrichtungen aufweist.

Eine Aufgabe der vorliegenden Erfindung besteht darin, eine Vorrichtung zur Behandlung von Atemschutzmasken anzugeben, welche eine unkomplizierte und schonende Aufnahme der jeweiligen Atemschutzmaske ermöglicht und zusätzlich eine gute Erreichbarkeit speziell der Maskenaußenseite für einen Sprühstrahl sicherstellt.

Erfindungsgemäß wird diese Aufgabe mittels einer im Folgenden mitunter kurz als Aufnahmegestell bezeichneten Vorrichtung zur Behandlung von Atemschutzmasken mit den Merkmalen des Anspruchs 1 gelöst. Dazu ist bei einer solchen Vorrichtung mit mehreren, um eine gemeinsame Achse drehbaren Haltevorrichtungen für jeweils eine Atemschutzmaske vorgesehen, dass jede Haltevorrichtung eine Trägerplatte und einen U-förmigen Haltebügel umfasst, wobei der U-förmige Haltebügel am freien Ende in Richtung auf die Trägerplatte abgewinkelt ist, wobei auf der Trägerplatte eine Atemschutzmaske platzierbar ist, wobei der U-förmige Haltebügel relativ zu der Trägerplatte schwenkbeweglich ist und mittels des U-förmigen Haltebügels eine auf der Trägerplatte platzierte Atemschutzmaske fixierbar ist.

Bei einem Verfahren zur Verwendung einer solchen Vorrichtung gemäß Anspruch 9 oder einer im Folgenden beschriebenen Ausführungsform einer solchen Vorrichtung wird in zumindest einer Haltevorrichtung eine Atemschutzmaske angebracht, indem die Atemschutzmaske auf der Trägerplatte der Haltevorrichtung platziert wird und durch Verschwenken des Haltebügels in eine Halteposition auf der Trägerplatte fixiert wird.

Ein Vorteil der Erfindung besteht darin, dass das gegenständliche Aufnahmegestell besonders einfach und intuitiv sowie ohne Gefahr von Fehlbedienungen zu bestücken und zu verwenden ist. Eine einzelne Atemschutzmaske ist sehr schnell auf der jeweiligen Trägerplatte platzierbar und dort mittels des Haltebügels fixierbar. Die Maskenaußenseite bleibt dabei gut zugänglich für den Sprühstrahl und die Maskeninnenseite ist gut zugänglich für ein Reinigungsmittel. Mittels des Aufnahmegestells ist eine Reinigung einer einzelnen Atemschutzmaske oder eine gleichzeitige Reinigung einer Vielzahl von Atemschutzmasken möglich. Neben einer Reinigung ist eine weitere Behandlung der oder jeder Atemschutzmaske möglich, zum Beispiel eine Behandlung in Form einer Trocknung. Deshalb wird die hier vorgeschlagene Vorrichtung auch als Vorrichtung zur Behandlung von Atemschutzmasken bezeichnet. Eine Reinigung ist nur eine Möglichkeit einer mittels der Vorrichtung erfolgenden Behandlung. Alternative oder weitere Behandlungsmöglichkeiten, also anstelle einer Reinigung oder zusätzlich zu einer Reinigung, sind dabei stets mitzulesen.

Vorteilhafte Ausgestaltungen der Erfindung sind Gegenstand der Unteransprüche. Dabei verwendete Rückbeziehungen weisen auf die weitere Ausbildung des Gegenstandes des Hauptanspruches durch die Merkmale des jeweiligen Unteranspruches hin. Des Weiteren ist im Hinblick auf eine Auslegung der Ansprüche sowie der Beschreibung bei einer näheren Konkretisierung eines Merkmals in einem nachgeordneten Anspruch davon auszugehen, dass eine derartige Beschränkung in den jeweils vorangehenden Ansprüchen sowie einer allgemeineren Ausführungsform des gegenständlichen Aufnahmegestells für Atemschutzmasken nicht vorhanden ist. Jede Bezugnahme in der Beschreibung auf Aspekte nachgeordneter Ansprüche ist demnach auch ohne speziellen Hinweis ausdrücklich als Beschreibung optionaler Merkmale zu lesen.

Bei einer Ausführungsform des Aufnahmegestells ist der oder jeder Haltebügel in einer ersten Schwenkposition (Halteposition) und in einer zweiten Schwenkposition (Entnahmeposition) lösbar arretierbar, wobei in der Halteposition der Haltebügel mit Abstand von der Oberfläche der Trägerplatte parallel oder zumindest im Wesentlichen parallel zur Oberfläche der Trägerplatte orientiert ist und mittels des Haltebügels in der Halteposition eine auf der zugehörigen Trägerplatte platzierte Atemschutzmaske fixierbar ist und wobei in der Entnahmeposition eine Atemschutzmaske auf der Trägerplatte platzierbar ist oder eine auf der Trägerplatte platzierte Atemschutzmaske aus der Vorrichtung entnehmbar ist. Durch einfaches Öffnen und Schließen des Haltebügels - Verschwenken des Haltebügels zwischen der Entnahmeposition und der Halteposition - wird eine Haltevorrichtung geöffnet und für die Aufnahme einer Atemschutzmaske vorbereitet bzw. die Haltevorrichtung geschlossen und eine auf der Trägerplatte platzierte Atemschutzmaske dort in einer für die jeweilige Behandlung, insbesondere Reinigung, ausreichenden Art und Weise fixiert, also in einer Art und Weise fixiert, welche gewährleistet, dass sich die Atemschutzmaske während des Behandlungs- oder Reinigungsvorgangs nicht aus der Haltevorrichtung löst.

Bei einer besonderen Ausführungsform des Aufnahmegestells wird der Haltebügel in der Halteposition mittels eines Hebelmechanismus und aufgrund der Federkraft eines an dem Hebelmechanismus angreifenden Federelements, insbesondere einer Zugfeder, auf eine auf der Trägerplatte platzierte Atemschutzmaske gedrückt. In der Entnahmeposition wird der Haltebügel gegen die Federkraft derselben Zugfeder gehalten.

Bei einer weiteren Ausführungsform des Aufnahmegestells ist der Haltebügel mittels eines in eine Kulisse eingreifenden Druckstücks in der Halteposition und in der Entnahmeposition lösbar arretierbar. Bevorzugt weist die Kulisse ein Langloch für die Arretierung des Haltebügels in der Halteposition sowie ein Loch für die Arretierung des Haltebügels in der Entnahmeposition auf. Das Langloch erlaubt auf der Halteposition noch eine gewisse Beweglichkeit (Schwenkbeweglichkeit) des Haltebügels. Im Rahmen dieser Beweglichkeit kann sich der Haltebügel aufgrund der Federkraft des an dem Hebelmechanismus angreifenden Federelements optimal an die Oberfläche der auf der Trägerplatte zu fixierenden Atemschutzmaske anlegen.

Bei einer besonderen Ausführungsform des Aufnahmegestells ist der Trägerplatte einer Haltevorrichtung ein Kleinteilebehälter zugeordnet. Dies erlaubt die Reinigung von Kleinteilen, wie zum Beispiel Federn oder Ventilscheiben, zusammen mit einer Atemschutzmaske. Indem der Kleinteilebehälter einer Trägerplatte zugeordnet ist, auf welcher die Atemschutzmaske platzierbar ist, ist die Zusammengehörigkeit von in dem Kleinteilebehälter befindlichen Kleinteilen und der Atemschutzmaske jederzeit erkennbar.

Besonders einfach ist der Kleinteilebehälter mit einer Haltevorrichtung kombinierbar, wenn die Trägerplatte Kleinteilebehälterrastausnehmungen umfasst, mittels derer der Kleinteilebehälter lösbar mit der Trägerplatte verbindbar ist. Dann kann der Kleinteilebehälter auch einzeln, insbesondere mit darin befindlichen Kleinteilen, entnommen werden. Darüber hinaus kann der Kleinteilebehälter leicht ersetzt und - falls notwendig - leicht separat gereinigt werden.

Bei einer weiteren Ausführungsform des Aufnahmegestells weist jede Trägerplatte eine Möglichkeit zum Einhaken von zum Beispiel langer Bebänderung oder dem Trageriemen auf. Die Standardbebänderung hängt frei unter der Trägerplatte und kann sich bei der Rotation des Gestells nicht mit anderen Teilen des Aufnahmegestells oder Düsen oder dergleichen verhaken.

Bei einer weiteren Ausführungsform des Aufnahmegestells ist der Haltebügel in einer Art und Weise geformt, welche gewährleistet, dass dieser die jeweilige Atemschutzmaske an deren Scheibenrahmen an vier Punkten berührt und mit einer über die Zugfeder bestimmten Kraft auf die Trägerplatte drückt. Durch die gebogene Form des Bügels wird sichergestellt, dass dieser nicht mit der teilweise auch gebogenen Maskenscheibe in Berührung kommt.

Bei einer nochmals weiteren Ausführungsform des Aufnahmegestells sind die Haltevorrichtungen an einem um seine Längsachse drehbaren, zylindrischen Zentralkörper des Aufnahmegestells angebracht. Durch Drehung des Zentralkörpers werden alle Haltevorrichtungen bewegt und gelangen im Zuge der Drehung zum Beispiel in ein Tauchbad, in den Bereich eines mittels zumindest einer Düse versprühten Reinigungsmittels, in den Bereich eines Gebläses oder einer Heizeinrichtung und so weiter.

Bei einer besonderen Ausführungsform eines Aufnahmegestells mit drehbarem Zentralkörper weisen die Trägerplatten der Haltevorrichtungen an dem Zentralkörper mit ihren freien Enden radial oder zumindest im Wesentlichen radial nach außen und eine Fläche der Trägerplatten ist parallel oder zumindest im Wesentlichen parallel zur Längsachse des Zentralkörpers orientiert. Bei einer solchen Orientierung kann eine Haltevorrichtung und jede Haltevorrichtung besonders einfach bestückt werden. Der Zentralkörper rotiert um eine horizontale oder im Wesentlichen horizontale Achse. Dies bedeutet, dass es für jede Trägerplatte eine Rotationslage des Zentralkörpers gibt, bei welcher die Fläche der Trägerplatte sowohl mit ihrer radialen Hauptachse wie auch mit ihrer parallel zur Drehachse des Zentralkörpers verlaufenden Hauptachse horizontal orientiert ist. Bei einer solchen Orientierung des Zentralkörpers kann die jeweilige Trägerplatte deshalb einfach bestückt werden, weil die Atemschutzmaske im Grunde auf der Trägerplatte abgelegt werden kann und nach dem Ablegen mittels des Haltebügels fixiert wird. Der Benutzer hat dann nach dem Ablegen der Atemschutzmaske die Hände frei, um den Haltebügel zu betätigen, ohne dass die Notwendigkeit besteht, die Atemschutzmaske mit zumindest einer Hand festhalten zu müssen, bis die Fixierung durch den Haltebügel gegeben ist.

Nachfolgend wird ein Ausführungsbeispiel der Erfindung anhand von Zeichnungen näher erläutert. Einander entsprechende Gegenstände oder Elemente sind in allen Figuren mit den gleichen Bezugszeichen versehen.

Das Ausführungsbeispiel ist nicht als Einschränkung der Erfindung zu verstehen. Vielmehr sind im Rahmen der vorliegenden Offenbarung Abänderungen und Modifikationen möglich, insbesondere solche Varianten und Kombinationen, die zum Beispiel durch Kombination oder Abwandlung von einzelnen in Verbindung mit den im allgemeinen oder speziellen Beschreibungsteil beschriebenen sowie in den Ansprüchen und/oder der Zeichnung enthaltenen Merkmalen für den Fachmann im Hinblick auf die Lösung der Aufgabe entnehmbar sind und durch kombinierbare Merkmale zu einem neuen Gegenstand führen.

Es zeigen:
- Figur 1: eine Vorrichtung zur Behandlung von Atemschutzmasken,
- Figur 2: in einer vergrößerten Ansicht mehrere Haltevorrichtungen der Vorrichtung gemäß Figur 1 mit jeweils einer Atemschutzmaske,
- Figur 3: eine Draufsicht auf eine Trägerplatte einer Haltevorrichtung,
- Figur 4: und
- Figur 5: die Haltevorrichtung mit einem in eine Halteposition bzw. in eine Entnahmeposition verschwenkten Haltebügel,
- Figur 6: den Haltebügel und einen den Haltebügel tragenden Haltewinkel in unterschiedlichen Ansichten sowie
- Figur 7: eine Draufsicht auf eine Haltevorrichtung mit einer auf deren Trägerplatte platzierten und mittels des Haltebügels fixierten Atemschutzmaske.

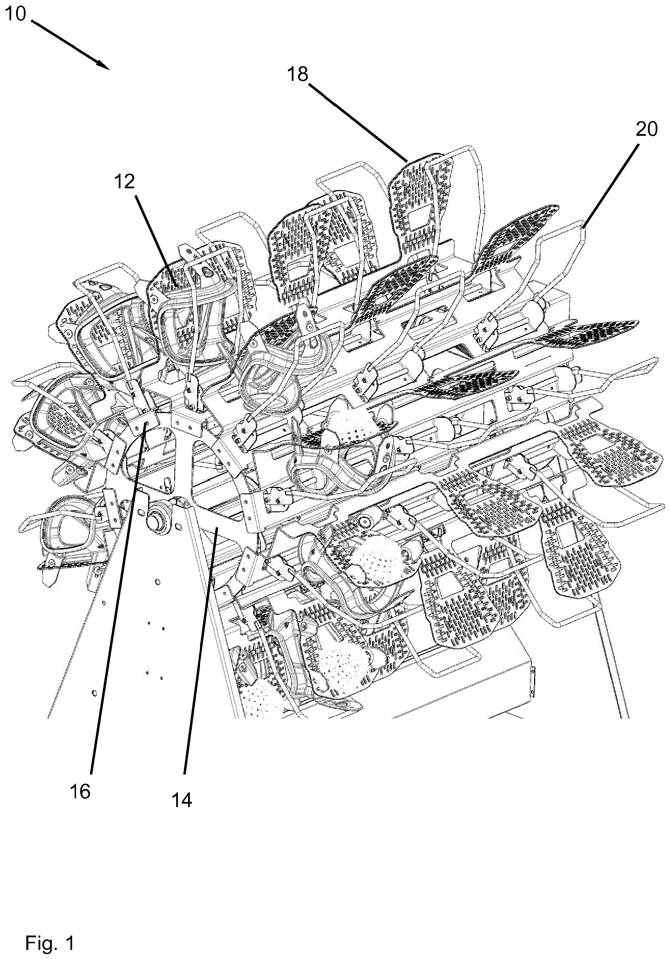

Die Darstellung in Figur 1 zeigt beispielhaft eine Ausführungsform einer im Folgenden mitunter kurz als Aufnahmegestell 10 bezeichneten Vorrichtung 10 zur Behandlung von Atemschutzmasken 12. Das Aufnahmegestell 10 umfasst einen um eine Mittellängsachse drehbaren Zentralkörper 14. Der Zentralkörper 14 wird dafür in einem die Drehachse bestimmenden Traggestell gehalten. Bei der gezeigten Ausführungsform ist der Zentralkörper 14 um eine horizontale oder zumindest im Wesentlichen horizontale Achse drehbar. Das Aufnahmegestell 10 ist zur Verwendung in einer grundsätzlich an sich bekannten und hier nicht gezeigten Reinigungskabine bestimmt.

Der Zentralkörper 14 hat bei der gezeigten Ausführungsform eine zylindrische Form. Der Zentralkörper 14 weist im Bereich von dessen Mantelfläche mehrere gleichmäßig beabstandete und parallel zur Mittellängsachse ausgerichtete Träger 16 auf. Von jedem Träger 16 geht - insbesondere einstückig - zumindest eine als Aufnahmeträger für eine Atemschutzmaske 12 (Maskenträger) fungierende Trägerplatte 18 aus. Bei der gezeigten Ausführungsform gehen eine Mehrzahl von Trägerplatten 18 von jeweils einem Träger 16 aus.

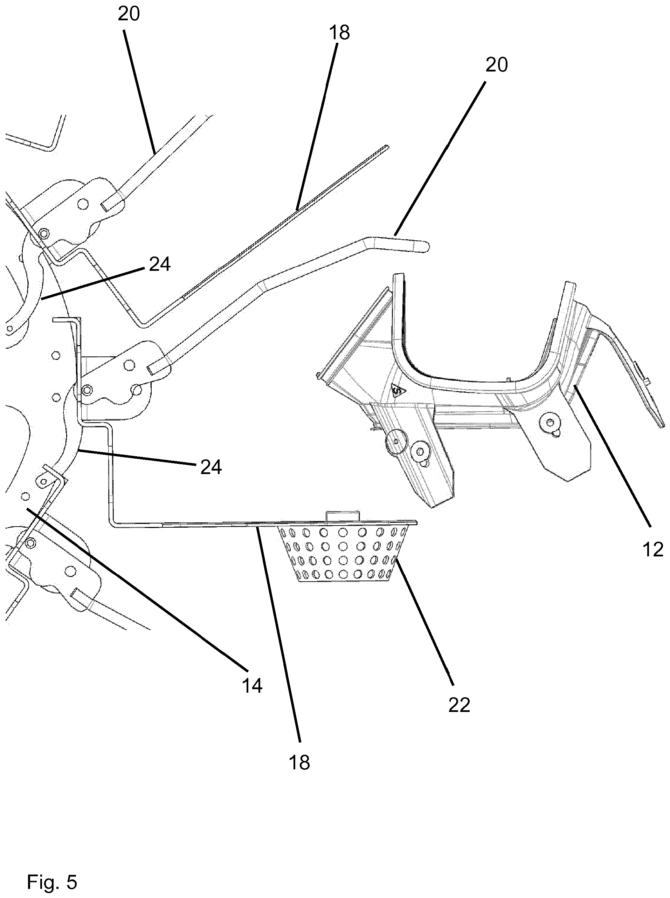

Bezogen auf den Zentralkörper 14 und dessen Mittellängsachse weist jede Trägerplatte 18 radial oder zumindest im Wesentlichen radial nach außen (eine Hauptachse der durch die Trägerplatte 14 definierten Fläche weist radial oder im Wesentlichen radial nach außen; die andere Hauptachse dieser Fläche ist parallel oder im Wesentlichen parallel zur Mittellängsachse/Drehachse des Zentralkörpers 14). Jeder Trägerplatte 18 ist ein Haltebügel 20 zugeordnet. Jeweils eine Trägerplatte 18 und der dieser zugeordnete Haltebügel 20 bilden zusammen eine Haltevorrichtung für eine Atemschutzmaske 12. Unter jeder Trägerplatte 18 kann ein Kleinteilebehälter 22 (Fig. 5) rastend angebracht werden.

Figur 1 zeigt eine Situation mit einem teilweise mit Atemschutzmasken 12 bestückten Aufnahmegestell 10 und nur in einem Teil der Haltevorrichtungen befindet sich eine dort mittels des Haltebügels 20 auf der jeweiligen Trägerplatte 18 fixierte Atemschutzmaske 12, während andere Haltevorrichtungen unbelegt sind.

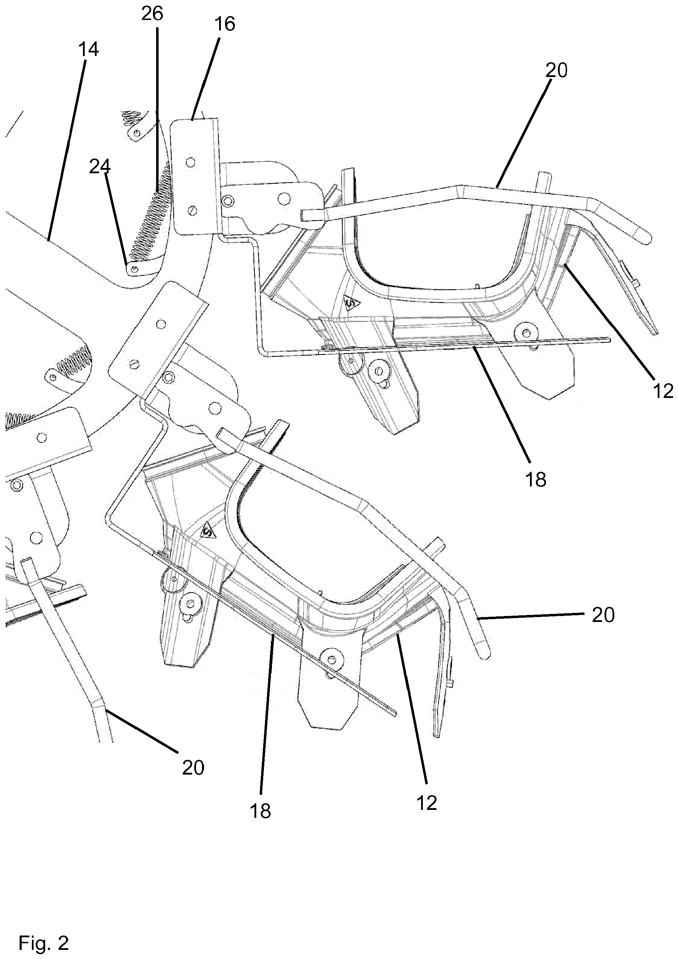

Die Darstellung in Figur 2 zeigt - bei einer Blickrichtung parallel zur Mittellängsachse des Zentralkörpers 14 - eine vergrößerte Darstellung eines Teils des Aufnahmegestells 10 gemäß Figur 1. In oberen Bildbereich ist eine Haltevorrichtung mit einer Atemschutzmaske 12 gezeigt. Aufgrund der Rotationslage des Zentralkörpers 14 befinden sich diese Haltevorrichtung und deren Trägerplatte 18 in einer "3 Uhr-Position", d.h. die Trägerplatte 18 ist horizontal oder im Wesentlich horizontal orientiert. Haltevorrichtungen in dieser Position lassen sich besonders einfach bestücken.

Die Atemschutzmaske 12 liegt mit ihrer Dichtseite auf der bei der gezeigten Rotationslage des Zentralkörpers 14 horizontal ausgerichteten Trägerplatte 18 und ist mittels des der Trägerplatte 18 zugeordneten Haltebügels 20 fixiert. Der Haltebügel 20 fixiert eine auf einer Trägerplatte 18 platzierte Atemschutzmaske 12 in jeder Rotationslage des Zentralkörpers 14 und insoweit zeigt die Darstellung in Figur 2 im unteren Bildbereich eine weitere Haltevorrichtung in einer anderen Rotationslage und eine dort fixierte Atemschutzmaske 12. Die jeweiligen Kleinteilebehälter 22 (hier nicht gezeigt) sind optional unter der Trägerplatte 18 angebracht.

Jeder Haltebügel 20 ist um eine Schwenkachse verschwenkbar. Der Haltebügel 20 erstreckt sich auf einer Seite der Schwenkachse. Auf der anderen Seite geht der Haltebügel 20 in einen Hebel 24 über, an dessen freiem Ende eine Zugfeder 26 angreift, welche an ihrem anderen Ende am Zentralkörper 14 angreift. Mittels der Federkraft der Zugfeder 26 wird der Haltebügel 20 in der die jeweilige Atemschutzmaske 12 auf der Trägerplatte 18 fixierenden Position gehalten, so wie dies in Figur 2 gezeigt ist.

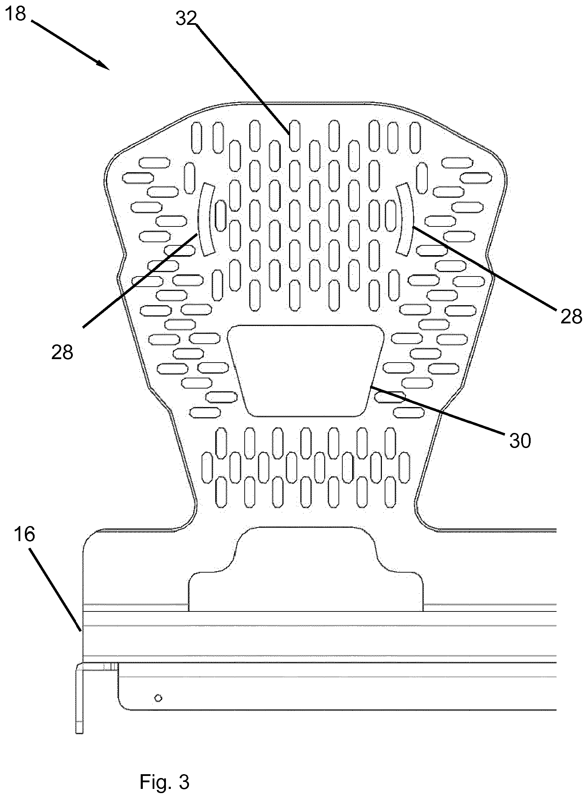

Die Darstellung in Figur 3 zeigt eine Draufsicht auf eine einzelne Trägerplatte 18. Hier sind zur insbesondere zentralen Anordnung des Kleinteilebehälters 22 bestimmte Ausnehmungen (Kleinteilebehälterrastausnehmungen) 28 erkennbar. Mittels dieser Ausnehmungen 28 wird der Kleinteilebehälter 22 lösbar, insbesondere rastend, mit der Trägerplatte 18 verbunden. Neben diesen Kleinteilebehälterrastausnehmungen 28 befinden sich in der Fläche der Trägerplatte 18 weitere Ausnehmungen 30, 32, welche gewährleisten, dass auch die Innenseite einer auf der Trägerplatte 18 platzierten Atemschutzmaske 12 für ein jeweiliges Reinigungsmittel, zum Beispiel ein mittels einer oder mehrerer Düsen verteiltes Reinigungsmittel, gut erreichbar ist.

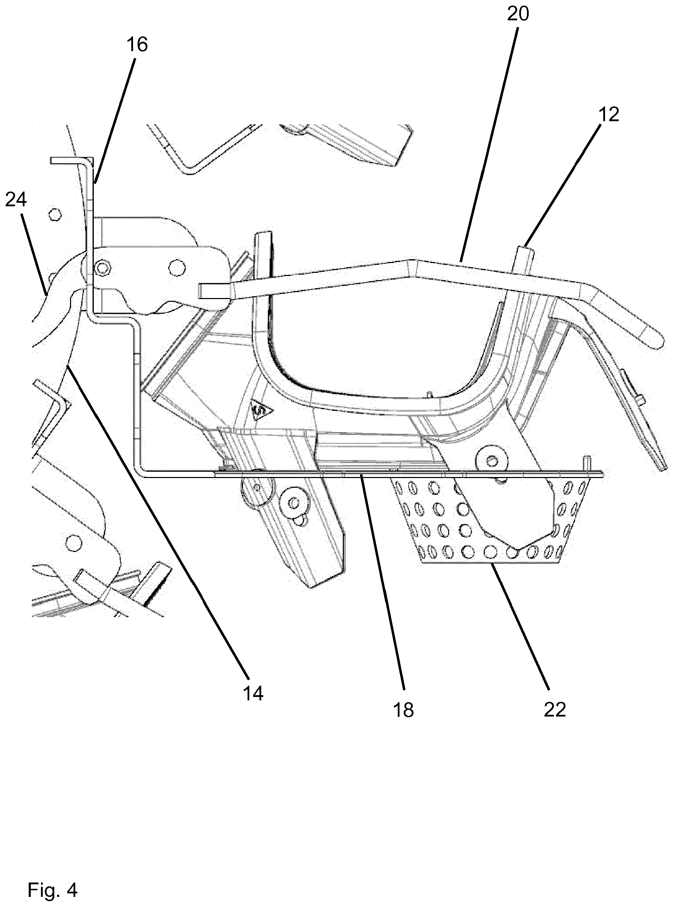

Der zur Fixierung einer auf einer Trägerplatte 18 platzierten Atemschutzmaske 12 bestimmte Haltebügel 20 ist schwenkbeweglich am Träger 16 angebracht. Dazu zeigt die Darstellung in Figur 4 eine mittels des Haltebügels 20 auf der Trägerplatte 18 fixierte Atemschutzmaske 12. Der Haltebügel 20 ist dabei parallel oder im Wesentlichen parallel zur Trägerplatte 18 orientiert und in dieser Orientierung arretiert. Die Arretierung erfolgt mittels der am Ende eines mit dem Haltebügel 20 verschwenkten Hebels 24 angreifenden Zugfeder 26 (Fig. 2).

Diese Position des Haltebügels 20 wird im Folgenden als Halteposition bezeichnet. Der Haltebügel 20 wird dabei federkraftbelastet auf die Atemschutzmaske 12 gedrückt. Die genaue Lage des Haltebügels 20 in der Halteposition ("untere Position") ergibt sich aufgrund der Maskengeometrie. D.h. der Haltebügel 20 passt sich in seiner Lage der Maskengeometrie an.

Zum Entnehmen der Atemschutzmaske 12 wird der Haltebügel 20 verschwenkt, zum Beispiel in eine Position, bei welcher Haltebügel 20 und Trägerplatte 18 einen Winkel von etwa 30° oder mehr einschließen. In der Darstellung in Figur 5 ist dies exemplarisch für einen relativ zur Trägerplatte 18 verschwenkten Haltebügel 20 gezeigt. Eine solche verschwenkte Position des Haltebügels 20 wird im Folgenden als Entnahmeposition bezeichnet.

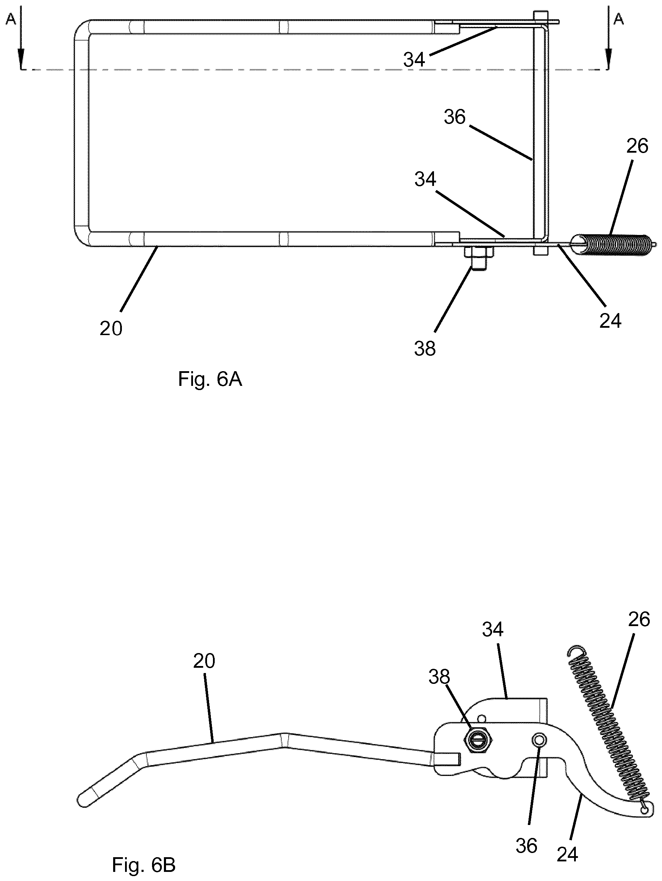

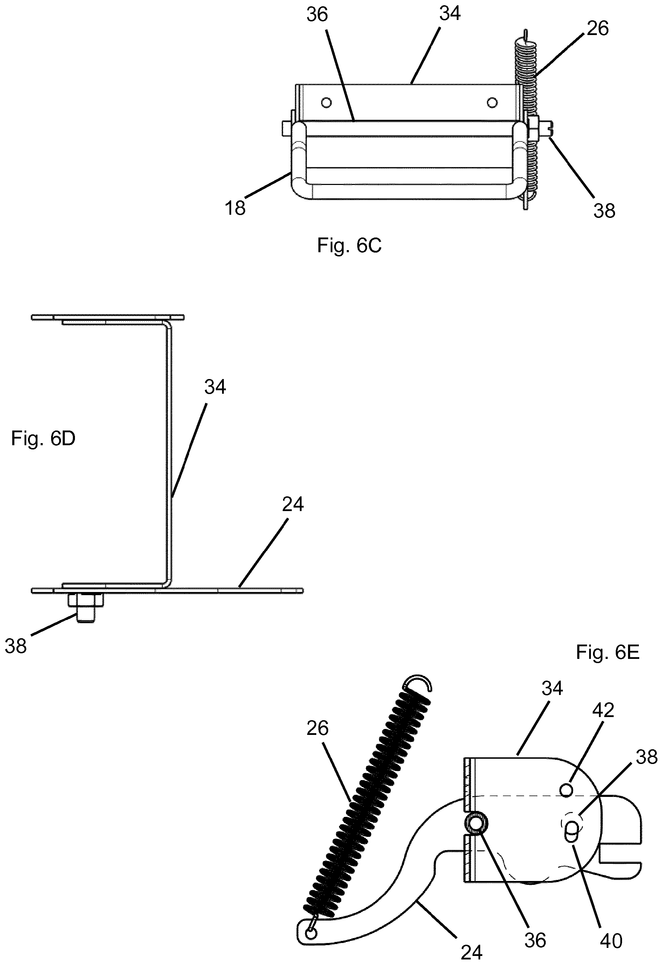

Die Darstellungen in Figur 6 (6A-6E) zeigen unterschiedliche Ansichten des Haltebügels 20 sowie eines Haltewinkels 34 für den Haltebügel 20. Figur 6A zeigt den Haltebügel 20 in einer Ansicht von oben. Dabei sind eine Schwenkachse 36 und der dem Haltebügel 20 an der Schwenkachse 36 gegenüberliegende Hebel 24 erkennbar. Die Darstellung in Figur 6B zeigt den Haltebügel 20 mit dem Hebel 24 in einer Ansicht von der Seite. Am freien Ende des Hebels 24 greift die bereits erwähnte Zugfeder 26 (siehe auch Figur 2) an. Die Darstellung in Figur 6C zeigt den Haltebügel 20 in einer Ansicht von vorn. In allen Darstellungen 6A, 6B und 6C ist ein am Haltebügel 20 angebrachtes Druckstück 38 erkennbar. Ein solches Druckstück 38 besteht bekanntlich aus einer Gewindehülse mit innenliegender Feder, die auf eine Kugel oder einen Druckstift wirkt. Das Druckstück 38 ist am Haltebügel 20 eingeschraubt und über das Gewinde in seiner Rastkraft einstellbar. Die Kugel oder der Druckstift des Druckstücks 38 greift bei entsprechenden Schwenkpositionen des Haltebügels 20 in eine die Halteposition und die Entnahmeposition definierende Kulisse im oder am Haltewinkel 34 ein, nämlich bei der gezeigten Ausführungsform in ein die Halteposition definierendes Langloch 40 oder ein die Entnahmeposition definierendes Loch 42 (jeweils im Haltewinkel 34).

Die Darstellungen in Figur 6D und 6E zeigen den Haltewinkel 34 aus unterschiedlichen Richtungen. In der Darstellung in Figur 6D, die außerdem das Druckstück 38 am Haltebügel 20 und den Hebel 24 am Haltebügel, aber nicht die Schwenkachse 36 zeigt, wird deutlich, dass der Haltewinkel 34 seitlich zwei gegenüber einem Basisteil um 90° abgewinkelte Schenkel aufweist. Die Darstellung in Figur 6E zeigt entlang der Schnittlinie A-A in Fig. 6A den Blick auf eine der Innenflächen der seitlichen Schenkel des Haltewinkels 34. Dieser fungiert als Kulisse für das (durch den Schenkel verdeckte und entsprechend mit gestrichelten Linien angedeutete) Druckstück 38. Die mittige Ausnehmung an der (in der Darstellung zur Seite weisenden) Unterkante des Schenkels und eine entsprechende Ausnehmung am gegenüberliegenden Schenkel des Haltewinkels 34 nehmen die Schwenkachse 36 des Haltebügels 20 auf. Mit Abstand von der Position der Schwenkachse 36 (gleicher Abstand wie zwischen der Schwenkachse 36 und dem Druckstück 38 am Haltebügel 20) befinden sich in dem Schenkel das erwähnte Langloch 40 und das ebenfalls erwähnte Loch 42, in welche das Druckstück 38 in der Halteposition bzw. der Entnahmeposition eingreift.

Das Langloch 40 erlaubt eine gewisse Beweglichkeit des Haltebügels 20 in der Halteposition und definiert Endpunkte für die Schwenkposition des Haltebügels 20 in der Entnahmeposition. Die konkrete Schwenkposition des Haltebügels 20 ergibt sich üblicherweise aufgrund der auf der Trägerplatte 18 platzierten Atemschutzmaske 12, auf deren Oberfläche der Haltebügel 20 mittels der Federkraft der Zugfeder 26 gedrückt wird.

Das Druckstück 38 verhindert unerwünschtes Öffnen des Haltebügels 20, falls z.B. der Antrieb des Aufnahmegestells 10 "ruckt" oder falls bei Stillstand des Aufnahmegestells 10 viel Flüssigkeit in das Innere der Atemschutzmaske 12 gelangt. Der Bediener muss beim Öffnen des Haltebügels 20 entsprechend die Kraft aufwenden, welche erforderlich ist, um das Druckstück 38 außer Eingriff mit dem Langloch 40 zu bringen.

Beim Entnehmen der Atemschutzmaske 12 wird der Haltebügel 20 gegen die Federkraft der Zugfeder 26 verschwenkt, bis das Druckstück 38 in das Loch 42 eingreift. In dieser Position wird der Haltebügel 20 mittels des Druckstücks 38 arretiert und zum erneuten Schließen des Haltebügels 20 muss der Bediener die Kraft aufwenden, welche erforderlich ist, um das Druckstück 38 außer Eingriff mit dem Loch 42 zu bringen.

Die beiden in Figur 4 und Figur 5 gezeigten Positionen (Halteposition bzw. Entnahmeposition) sind Beispiele für die Endpositionen der Schwenkbeweglichkeit des Haltebügels 20. Zur Arretierung des Haltebügels 20 in jeweils einer solchen Endposition weist das Aufnahmegestell 10 als Mittel zur lösbaren Arretierung des Haltebügels 20 das erwähnte Druckstück 38 auf. Andere Mittel zur rastenden Arretierung des Haltebügels 20 sind alternativ ebenfalls denkbar, zum Beispiel ein die Endposition der Schwenkbewegung definierender Anschlag und eine federnde Rastnase in einem Abstand von dem Anschlag.

Bei der gezeigten erfindungsgemäßen Ausführungsform ist der Haltebügel 20 am freien Ende in Richtung auf die Trägerplatte 18 abgewinkelt. Dies gewährleistet im Vergleich zum einem ansonsten ebenfalls denkbaren, nicht erfindungsgemäßen, nicht abgewinkelten Haltebügel 20 eine noch bessere Fixierung einer auf der Trägerplatte 18 platzierten Atemschutzmaske 12.

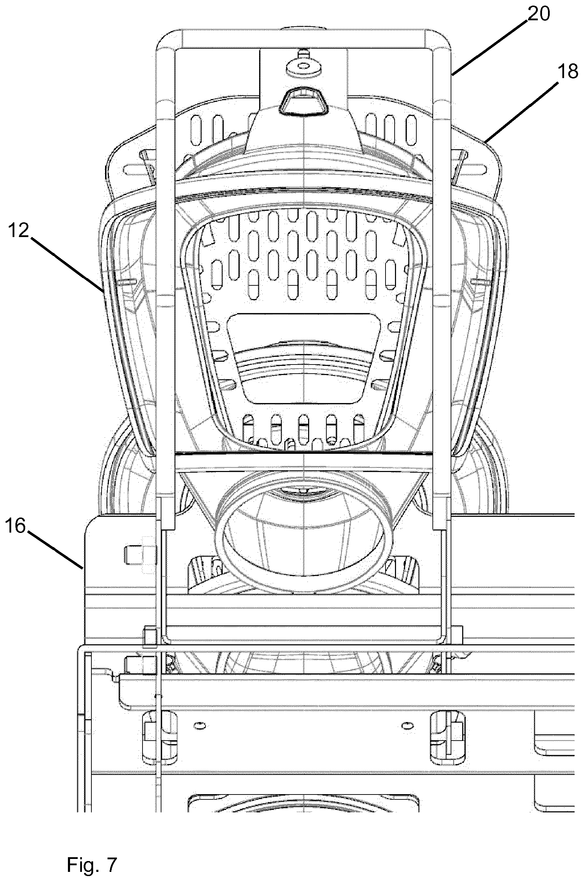

Figur 7 zeigt eine Draufsicht auf eine mit einer Atemschutzmaske 12 bestückte Haltevorrichtung. Die auf der Trägerplatte 18 platzierte Atemschutzmaske 12 ist mittels des Haltebügels 20 auf der Trägerplatte 18 fixiert. In der Draufsicht ist die bereits in Figur 6A erkennbare U-Form des Haltebügels 20 deutlich zu erkennen, wobei dessen freie Enden an dem Träger 16 gehalten werden. Mit seinen langen Schenkeln umgreift der Haltebügel 20 die Atemschutzmaske 12 beidseitig und liegt dabei auf dem stabilen Fensterrahmen der Atemschutzmaske 12 auf. Auf diese Weise wird verhindert, dass die Atemschutzmaske 12 durch den Haltebügel 20 beschädigt wird.

Bei einer besonderen Ausführungsform des gegenständlichen Aufnahmegestells 10 ist neben der Möglichkeit zur Aufnahme einer Mehrzahl von Atemschutzmasken 12 auch die Möglichkeit zur Aufnahme von Lungenautomaten vorgegeben. Das Aufnahmegestell 10 weist in der in Figur 1 dargestellten Ausführungsform erkennbar in Umfangsrichtung des Zentralkörpers 14 in mehreren parallelen Ebenen eine Vielzahl von Haltevorrichtungen mit jeweils einer Trägerplatte 18 und einem Haltebügel 20 auf. Eine oder mehrere derartige Ebenen können zur Aufnahme von Lungenautomaten eingerichtet werden. Dann ist mittels des hier vorgeschlagenen Aufnahmegestells 10 auch eine gleichzeitige Reinigung von Atemschutzmasken 12 und Lungenautomaten möglich.

Einzelne im Vordergrund stehende Aspekte der hier eingereichten Beschreibung lassen sich damit kurz wie folgt zusammenfassen: Angegeben wird eine Vorrichtung 10 - Aufnahmegestell - zur Behandlung von Atemschutzmasken 12 mit mehreren, um eine gemeinsame Achse drehbaren Haltevorrichtungen 18, 20 für jeweils eine Atemschutzmaske 12, wobei jede Haltevorrichtung 18, 20 eine Trägerplatte 18 und einen U-förmigen Haltebügel 20 umfasst, wobei der U-förmige Haltebügel 20 am freien Ende in Richtung auf die Trägerplatte 18 abgewinkelt ist, wobei auf der Trägerplatte 18 eine Atemschutzmaske 12 platzierbar ist und wobei der U-förmige Haltebügel 20 relativ zu der Trägerplatte 18 schwenkbeweglich ist sowie mittels des U-förmigen Haltebügels 20 eine auf der Trägerplatte 18 platzierte Atemschutzmaske 12 fixierbar ist.

### BEZUGSZEICHENLISTE

- 10: Aufnahmegestell; Vorrichtung zur Behandlung von Atemschutzmasken
- 12: Atemschutzmaske
- 14: Zentralkörper
- 16: Träger
- 18: Trägerplatte
- 20: Haltebügel
- 22: Kleinteilebehälter
- 24: Hebel
- 26: Zugfeder
- 28: Kleinteilebehälterrastausnehmung
- 30: Ausnehmung
- 32: Ausnehmung
- 34: Haltewinkel
- 36: Schwenkachse
- 38: Druckstück
- 40: Langloch
- 42: Loch

## Patentansprüche

1. Vorrichtung (10) zur Behandlung von Atemschutzmasken (12) mit mehreren, um eine gemeinsame Achse drehbaren Haltevorrichtungen (18, 20) für jeweils eine Atemschutzmaske (12),
wobei jede Haltevorrichtung (18, 20) eine Trägerplatte (18) umfasst, wobei auf der Trägerplatte (18) eine Atemschutzmaske (12) platzierbar ist und
wobei eine auf der Trägerplatte (18) platzierte Atemschutzmaske (12) fixierbar ist,
**dadurch gekennzeichnet,**
**dass** jede Haltevorrichtung (18, 20) einen U-förmigen Haltebügel (20) umfasst,
wobei der U-förrmige Haltebügel (20) relativ zu der Trägerplatte (18) schwenkbeweglich ist,
wobei mittels des U-förmigen Haltebügels (20) eine auf der Trägerplatte (18) platzierte Atemschutzmaske (12) fixierbar ist und
wobei der U-förmige Haltebügel (20) am freien Ende in Richtung auf die Trägerplatte (18) abgewinkelt ist.

2. Vorrichtung (10) nach Anspruch 1,
wobei der U-förmige Haltebügel (20) in einer ersten Schwenkposition und in einer zweiten Schwenkposition lösbar arretierbar ist,
wobei in der ersten, als Halteposition fungierenden Schwenkposition der U-förmige Haltebügel (20) mit Abstand von der Oberfläche der Trägerplatte (18) parallel zur Oberfläche der Trägerplatte (18) orientiert ist und mittels des U-förmigen Haltebügels (20) in der Halteposition eine auf der zugehörigen Trägerplatte (18) platzierte Atemschutzmaske (12) fixierbar ist und
wobei in der zweiten, als Entnahmeposition fungierenden Schwenkposition eine Atemschutzmaske (12) auf der Trägerplatte (18) platzierbar ist oder eine auf der Trägerplatte (18) platzierte Atemschutzmaske (12) aus der Vorrichtung (10) entnehmbar ist.

3. Vorrichtung (10) nach Anspruch 2, wobei der U-förmige Haltebügel (20) mittels eines in eine Kulisse eingreifenden Druckstücks (38) in der Halteposition und in der Entnahmeposition lösbar arretierbar ist.

4. Vorrichtung (10) nach Anspruch 3,
wobei die Kulisse ein Langloch (40) für die Arretierung des U-förmigen Haltebügels (20)
in der Halteposition und ein Loch (42) für die Arretierung des U-förmigen Haltebügels (20) in der Entnahmeposition aufweist.

5. Vorrichtung (10) nach einem der vorangehenden Ansprüche,
wobei der Trägerplatte (18) einer Haltevorrichtung (18, 20) ein Kleinteilebehälter (22) zugeordnet ist.

6. Vorrichtung (10) nach Anspruch 5,
wobei die Trägerplatte (18) Kleinteilebehälterrastausnehmungen (28) zum lösbaren Anbringen des Kleinteilebehälters (22) an der Trägerplatte (18) aufweist.

7. Vorrichtung (10) nach einem der vorangehenden Ansprüche,
wobei die Haltevorrichtungen (18, 20) an einem um seine Längsachse drehbaren, zylindrischen Zentralkörper (14) angebracht sind.

8. Vorrichtung (10) nach Anspruch 7,
wobei die Trägerplatten (18) der Haltevorrichtungen (18, 20) an dem Zentralkörper (14) mit ihren freien Enden radial nach außen weisen und eine Fläche der Trägerplatten (18) parallel zur Längsachse des Zentralkörpers (14) orientiert ist.

9. Verfahren zur Verwendung einer Vorrichtung (10) nach einem der vorangehenden Ansprüche, wobei in zumindest einer Haltevorrichtung (18, 20) eine Atemschutzmaske (12) angebracht wird, indem die Atemschutzmaske (12) auf der Trägerplatte (18) der Haltevorrichtung (18, 20) platziert wird und durch Verschwenken des U-förmigen Haltebügels (20) in eine Halteposition auf der Trägerplatte (18) fixiert wird.

## Claims

1. Device (10) for treating respiratory masks (12), having a plurality of holding devices (18, 20) which are rotatable about a common axis and are intended for one respiratory mask (12) each,
wherein each holding device (18, 20) comprises a carrier plate (18),
wherein a respiratory mask (12) can be placed on the carrier plate (18), and
wherein a respiratory mask (12) placed on the carrier plate (18) can be secured,
**characterized**
**in that** each holding device (18, 20) comprises a U-shaped holding clip (20),
wherein the U-shaped holding clip (20) is pivotable relative to the carrier plate (18),
wherein a respiratory mask (12) placed on the carrier plate (18) can be secured by means of the U-shaped holding clip (20), and
wherein the U-shaped holding clip (20) is angled at the free end in the direction of the carrier plate (18).

2. Device (10) according to Claim 1,
wherein the U-shaped holding clip (20) can be releasably locked in a first pivot position and in a second pivot position,
wherein, in the first pivot position acting as a holding position, the U-shaped holding clip (20) is oriented parallel to the surface of the carrier plate (18) at a distance from the surface of the carrier plate (18) and a respiratory mask (12) placed on the associated carrier plate (18) can be secured in the holding position by means of the U-shaped holding clip (20), and
wherein, in the second pivot position acting as a removal position, a respiratory mask (12) can be placed on the carrier plate (18) or a respiratory mask (12) placed on the carrier plate (18) can be removed from the device (10).

3. Device (10) according to Claim 2,
wherein the U-shaped holding clip (20) can be releasably locked in the holding position and in the removal position by means of a pressure piece (38) engaging in a guide slot.

4. Device (10) according to Claim 3,
wherein the guide slot has an elongated hole (40) for locking the U-shaped holding clip (20) in the holding position and a hole (42) for locking the U-shaped holding clip (20) in the removal position.

5. Device (10) according to one of the preceding claims,
wherein the carrier plate (18) of a holding device (18, 20) is assigned a small parts container (22).

6. Device (10) according to Claim 5,
wherein the carrier plate (18) has small parts container latching recesses (28) for releasably attaching the small parts container (22) to the carrier plate (18).

7. Device (10) according to one of the preceding claims,
wherein the holding devices (18, 20) are attached to a cylindrical central body (14) which is rotatable about its longitudinal axis.

8. Device (10) according to Claim 7,
wherein the carrier plates (18) of the holding devices (18, 20) face radially outwards with their free ends at the central body (14) and a surface of the carrier plates (18) is oriented parallel to the longitudinal axis of the central body (14).

9. Method for using a device (10) according to one of the preceding claims, wherein a respiratory mask (12) is mounted in at least one holding device (18, 20) by the respiratory mask (12) being placed on the carrier plate (18) of the holding device (18, 20) and being secured by pivoting of the U-shaped holding clip (20) into a holding position on the carrier plate (18).

## Revendications

1. Dispositif (10) pour le traitement de masques de protection respiratoire (12) muni de plusieurs dispositifs de maintien (18, 20), rotatifs autour d'un axe commun, chacun pour un masque de protection respiratoire (12),
chaque dispositif de maintien (18, 20) comprenant une plaque support (18),
un masque de protection respiratoire (12) pouvant être placé sur la plaque support (18), et
un masque de protection respiratoire (12) placé sur la plaque support (18) pouvant être fixé,
**caractérisé en ce que**
chaque dispositif de maintien (18, 20) comprend un étrier de maintien en forme de U (20),
l'étrier de maintien en forme de U (20) étant mobile en pivotement par rapport à la plaque support (18),
un masque de protection respiratoire (12) placé sur la plaque support (18) pouvant être fixé au moyen de l'étrier de maintien en forme de U (20), et
l'étrier de maintien en forme de U (20) étant coudé à l'extrémité libre dans la direction de la plaque support (18).

2. Dispositif (10) selon la revendication 1, dans lequel l'étrier de maintien en forme de U (20) peut être bloqué de manière détachable dans une première position de pivotement et dans une deuxième position de pivotement, dans la première position de pivotement, fonctionnant en tant que position de maintien, l'étrier de maintien en forme de U (20) étant orienté à une distance de la surface de la plaque support (18) parallèlement à la surface de la plaque support (18), et un masque de protection respiratoire (12) placé sur la plaque support correspondante (18) pouvant être fixé au moyen de l'étrier de maintien en forme de U (20) dans la position de maintien, et
dans la deuxième position de pivotement, fonctionnant en tant que position de retrait, un masque de protection respiratoire (12) pouvant être placé sur la plaque support (18) ou un masque de protection respiratoire (12) placé sur la plaque support (18) pouvant être retiré du dispositif (10).

3. Dispositif (10) selon la revendication 2, dans lequel l'étrier de maintien en forme de U (20) peut être bloqué de manière détachable dans la position de maintien et dans la position de retrait au moyen d'un élément de pression (38) pénétrant dans une coulisse.

4. Dispositif (10) selon la revendication 3, dans lequel la coulisse comprend un orifice longitudinal (40) pour le blocage de l'étrier de maintien en forme de U (20) dans la position de maintien et un orifice (42) pour le blocage de l'étrier de maintien en forme de U (20) dans la position de retrait.

5. Dispositif (10) selon l'une quelconque des revendications précédentes, dans lequel un contenant de petites pièces (22) est associé à la plaque support (18) d'un dispositif de maintien (18, 20).

6. Dispositif (10) selon la revendication 5, dans lequel la plaque support (18) comprend des évidements d'encliquetage de contenant de petites pièces (28) pour la disposition détachable du contenant de petites pièces (22) sur la plaque support (18).

7. Dispositif (10) selon l'une quelconque des revendications précédentes, dans lequel les dispositifs de maintien (18, 20) sont disposés sur un corps central cylindrique (14) rotatif autour de son axe longitudinal.

8. Dispositif (10) selon la revendication 7, dans lequel les plaques supports (18) des dispositifs de maintien (18, 20) sont dirigées radialement vers l'extérieur avec leurs extrémités libres sur le corps central (14) et une surface des plaques supports (18) est orientée parallèlement à l'axe longitudinal du corps central (14).

9. Procédé d'utilisation d'un dispositif (10) selon l'une quelconque des revendications précédentes, dans lequel un masque de protection respiratoire (12) est disposé dans au moins un dispositif de maintien (18, 20), par le fait que le masque de protection respiratoire (12) est placé sur la plaque support (18) du dispositif de maintien (18, 20) et fixé dans une position de maintien sur la plaque support (18) par pivotement de l'étrier de maintien en forme de U (20).
